# EUROPEAN PATENT SPECIFICATION

(11) **EP 1 372 498 B1**
(45) Date of publication and mention of the grant of the patent: **26.07.2006**
(21) Application number: 02717605.6
(22) Date of filing: 11.03.2002
(51) Int. Cl.: A61B 17/34

(54) **EPIDURAL NEEDLE AND COMBINED SPINAL/EPIDURAL NEEDLE SET OF VARIABLE EXTENSION**
EPIDURALNADEL UND KOMBINIERTES SPINAL-, EPIDURALNADELSET VARIABLER EXTENSION
AIGUILLE PERIDURALE ET JEU D'AIGUILLE SPINALE/PERIDURALE COMBINEE A EXTENSION VARIABLE

(30) Priority: 30.03.2001 US 823199
(43) Date of publication of application: 02.01.2004
(73) Proprietor: Becton Dickinson and Company, Franklin Lakes, New Jersey 07417 (US)
(72) Inventor: MARSH, Ronald, W., Hackettstown, NJ 07840 (US); DAW, Sean, P., Chicago, IL 60647 (US)
(74) Representative: Ruffles, Graham Keith
(86) International application number: PCT/US2002/007366
(87) International publication number: WO 2002/078551

(56) References cited:
- EP-A- 0 691 139
- EP-A- 0 982 006

## Description

### Field of Invention

The present invention is generally related to the field of hypodermic needles and more specifically to hypodermic needles intended for administration and withdrawal of fluids to the spine of a patient.
An epidural needle according to the preamble of claim 1 is known from document EP-A-0982006.

### Background

Generally speaking, there are two basic techniques for introducing injectable medicament into the spinal area of a patient. The techniques both can be used to create spinal anesthesia, one being delivery of the medicament into the epidural space, "epidural," and the other, penetration of the dural membrane with delivery of the medicament into the subarachnoid space, "spinal" or "subarachnoid." The medicaments can be any type of liquid therapeutic material including antibiotics, steroids and the like, but generally are agents used for anesthesia and analgesia. When the liquid medicament is an anesthetic agent, a subarachnoid placement is recognized as providing a faster, more uniform distribution, but several major side effects may result from an improper subarachnoid placement. These side effects may include nerve damage, either from contact with the needle or from high local concentrations of the medicament, pooling or inadequate mixing of the medicament in the cerebrospinal fluid.

Delivery of the medicament into the subarachnoid space requires a penetration depth of several centimeters from the surface of the back. Puncture of the dural membrane for introduction of a needle or catheter with a large gauge needle may result in postoperative leakage of cerebrospinal fluid from the puncture site, often resulting in severe postoperative headaches. Thus, when puncture of the dural membrane is made with a needle, the smaller the size of the puncture the lower the probability of post-procedural leakage of cerebrospinal fluid. Small diameter needles of the length required to enter the subarachnoid space are quite flexible and as a result, difficult to accurately position when making penetrations to a depth of several centimeters. Practitioners have recognized the need to use a needle with sufficient stiffness to make the initial penetration and the need to use a small diameter needle for penetration of the dural membrane. This recognition has evolved into the use of an eight to ten centimeters long, larger diameter (ca. 16-18 gauge) introducer needle to enter the epidural space followed by the use of the bore of the introducer needle to place a longer, i.e., eleven to sixteen centimeters long, smaller diameter (ca. 22-28 gauge) spinal needle adjacent to and then to penetrate the dural membrane. The spinal needle is then used to administer a bolus of the anesthetic agent. The bolus results in rapid onset of anesthesia, and depending upon the placement and the amount administered, the effect may last several hours.

Correct placement and delivery of a subarachnoid medicament is recognized by practitioners as being one of the more technique and tactilely sensitive procedures currently practiced. There is considerable anatomical variation between patients related to the patient's size and weight. The practitioner generally positions the introducer needle between the vertebrae into the epidural space adjacent the dural membrane (dura), then advances the spinal needle through the dura membrane into the subarachnoid space. Accurately perceiving when the dura has been penetrated is often difficult for the practitioner. There are several widely practiced techniques to confirm that the needle has entered the subarachnoid space. Some practitioners depend upon feeling a "pop" as the spinal needle penetrates the dura. Many practitioners also often confirm that the subarachnoid space is penetrated by using the spinal needle to withdraw a sample of cerebrospinal fluid.

A survey of previous patent literature reports in this general area is found in U.S. Patent No. 5,085,631. The patent discloses a method for placement of a subarachnoid catheter that utilizes a three component apparatus comprising an outer needle, an inner needle and a catheter intermediate the two needles.

A recent U.S. Patent No. 5,312,375, discloses a set for spinal anesthesia that includes a spinal needle, a stylet, an introducer needle through which the spinal needle is introduced and a clamp for fixing the spinal needle to the introducer needle to stabilize the spinal needle. The patent teaches that the tube portion of the introducer needle protrudes proximally beyond the introducer needle hub so that a regulating device with a thumb screw or a toothed member can engage both the introducer needle and the spinal needle to fix the position of the spinal needle relative to the introducer needle. Generally, the introducer needle is an epidural needle. As disclosed in U. S. Patent No. 5,312,375, the introducer needle cannot function as a conventional epidural needle, because the fluid path of the epidural needle is not fluid tight to a fluid handling attachment at the hub of the needle.

U. S. Patent No. 5,584,820, discloses a variant of the regulating device disclosed in U.S. Patent No. 5,312,375 for adjusting the length of a combined spinal epidural needle and the method of practicing its use. The disclosed invention utilizes standard commercially available spinal and epidural needles, adding a fixture for preselecting the spinal needle projection with respect to the epidural needle when the spinal needle is coaxially placed within the epidural needle. While this regulating device and method may be quite useful in the practice of combined spinal epidural medication, the use of this device disclosed in U.S. Patent No. 5,584,820 adds an additional item to the procedure kit, and additional manipulations. Additionally, neither of these variants teach that the attachment between the spinal needle and the epidural needle forms a fluid tight seal, thus, leakage of medicament or cerebrospinal fluid between the spinal needle and the epidural needle may occur.

Subarachnoid placement of medicaments, if done properly, is recognized as desirable. Thus, a device and a method for its use that would minimize the size of the puncture of the dural membrane, allow accurate and controlled placement of a therapeutically effective amount of a medicament within the subarachnoid space, thereby reducing the potential for nerve damage, coupled with an ability to rapidly initiate and maintain a therapeutic level of the medicament for longer procedures would represent an advance to the medical arts. If such an epidural needle facilitated the subarachnoid placement and was fully functional as a standard epidural needle the art would be further advanced. A method and apparatus that addresses these needs constitute the present invention.

### Summary

An epidural needle of the present invention comprises the features defined in claim 1. It includes a hollow bore therethrough and is useful for releasably fixing a position of a spinal needle disposed within the bore of the epidural needle.

The epidural needle of the invention has an elongate tube defining a longitudinal axis having a proximal end, a distal end and an axial hollow bore having an inside diameter therethrough. The needle has a hub with a proximal end, a distal end and an open passageway having an inside diameter substantially similar to the hollow bore therethrough, the distal end of the hub being fixedly attached to the proximal end of the elongate tube so that the hollow bore of the elongate tube is in fluid communication and substantial axial alignment with the open passageway. The hub further has a cavity disposed between the proximal end and the distal end of the hub. There is a resilient member with an opening therethrough that has an inner diameter substantially similar to the inside diameter of the hollow bore disposed in the cavity so that the opening is substantially axially aligned and in fluid communication with the open passageway. The hub of the epidural needle of the invention has a clamp, preferably with a releasable latch disposed about the resilient member. The clamp is selectively movable between an open position wherein the inner diameter of the resilient member is substantially unaffected and a clamp position wherein the clamp causes a strain to the resilient member and thereby reduces the inner diameter of the opening through the resilient member. The preferred clamp also has a latch position where the latch releasably retains the clamp in the clamp position. It is to be understood that the clamp can be in substantially the same orientation when in the clamp position and the latch position Thus, a practitioner using the epidural needle of the invention to position a spinal needle with an outside diameter less than the inside diameter of the hollow tube may freely axially move the spinal needle within the hollow bore with respect to the epidural needle and fix a position of the spinal needle relative to the epidural needle by the reduction of the inner diameter opening through the resilient member to a diameter less than the outside diameter of the spinal needle by movement of the clamp to the clamp position and the latch position.

The epidural needle of the invention is easily manipulated by the practitioner to position the spinal needle. Additionally, the epidural needle of the invention is fully functional as a standard epidural needle since the fluid path from the needle bore to the hub is fluid tight. The projection of the spinal needle relative to the epidural needle is substantially infinitely variable within the full range of projection and, once the desired position is achieved, easily fixed by engaging the clamp on the resilient member. Until the clamp is engaged, a practitioner does not need to alter practices used with a standard epidural needle and a standard spinal needle. The engagement of the clamp to fix the position of the spinal needle relative to the epidural needle is facile and substantially intuitive. The invention provides an advance to the art of delivery of medicaments to the subarachnoid space.

### Brief Description of the Drawings

The epidural needle and kit shown in figures 1 to 7 does not fall under the scope of the appended claims, but serves only the better understanding of the invention.
Fig. 1 is a perspective view of an epidural needle in a kit including other elements;
Fig. 2 is partially exploded perspective view of the epidural needle of Fig. 1;
Fig. 3 is a side elevation view of Fig. 1 with a spinal needle positioned in the hollow bore of the epidural needle;
Fig. 4 is a horizontal cross-sectional view taken from Fig. 2 along the line 4-4;
Fig. 5A is a cross-sectional view of the epidural needle taken from Fig. 2 along the line 5A-5A;
Fig. 5B is a cross-sectional view of the epidural needle analogous to Fig. 5A, illustrating the clamp in the clamp/latch position;
Fig. 6 is a schematic cross-sectional view of a portion of the human spine penetrated by the epidural needle of Fig. 1;
Fig. 7 is a schematic cross-sectional view of the portion of the human spine illustrated in Fig. 6 with a spinal needle projecting a distance from the distal end of the epidural needle ;
Fig. 8 is a perspective view of an preferred embodiment of the clamp for the epidural needle according to an embodiment of the invention;
Fig. 9 is a cross-sectional view of the epidural needle according to an embodiment of the invention similar to Fig. 5A, showing the preferred embodiment of the clamp;
Fig. 10 is a cross-sectional view of the epidural needle according to an embodiment of the invention, analogous to Fig. 9, showing the preferred embodiment of the clamp in the clamp/latch position; and
Fig. 11 is a cross-sectional view of the epidural needle according to an embodiment of the invention, analogous to Fig. 9, showing the preferred embodiment of the clamp in the clamp position but with the push tab depressed to allow the clamp to be moved from the clamping position.

### Detailed Description

While this invention is satisfied by embodiments in many different forms, there is shown in the drawings and herein described in detail preferred embodiments of the invention with the understanding that the present disclosure is to be considered exemplary of the principles of the invention and is not intended to limit the invention to the embodiments illustrated. The scope of the invention is measured by the appended claims. For the purposes of this description of the present invention, the term "distal end" refers to the end of the assembly closest to the needle point and the patient, whereas the term "proximal end" refers to the end of the assembly furthest from the needle point and closest to the practitioner.

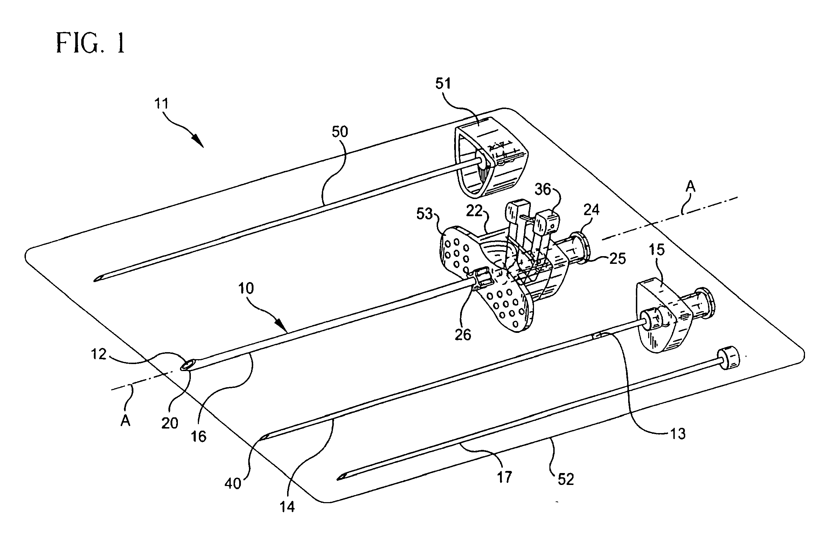

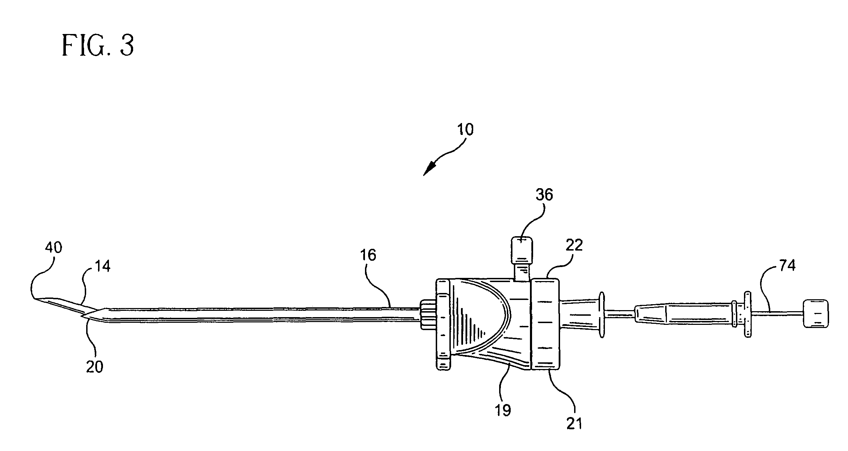

Referring to Figs. 1-11, an epidural needle 10 includes a hollow bore 12 therethrough and is useful for releasably fixing a position of a spinal needle 14 disposed within bore 12 of the epidural needle. Spinal needle 14 has a proximal hub 15. Epidural needle 10 has an elongate tube 16 defining a longitudinal axis "A" having a proximal end 18 , a distal end 20 and axial hollow bore 12 having an inside diameter "b" therethrough. Epidural needle 10 has a hub 22 with a proximal end 24, a distal end 26 and an open passageway 28 having an inside diameter substantially similar to inside diameter "b" of hollow bore 12 therethrough. Distal end 26 of hub 22 is fixedly attached to proximal end 18 of elongate tube 16 so that hollow bore 12 of elongate tube 16 is in fluid communication and substantial axial alignment with open passageway 28. Hub 22 further has a cavity 30 disposed between proximal end 24 and distal end 26 of the hub. There is a resilient member 32 with an opening 34 therethrough that has an inner diameter "d" substantially similar to inside diameter "b" of hollow bore 12 disposed in cavity 30 so that opening 34 in the resilient member is substantially axially aligned and in fluid communication with open passageway 28.

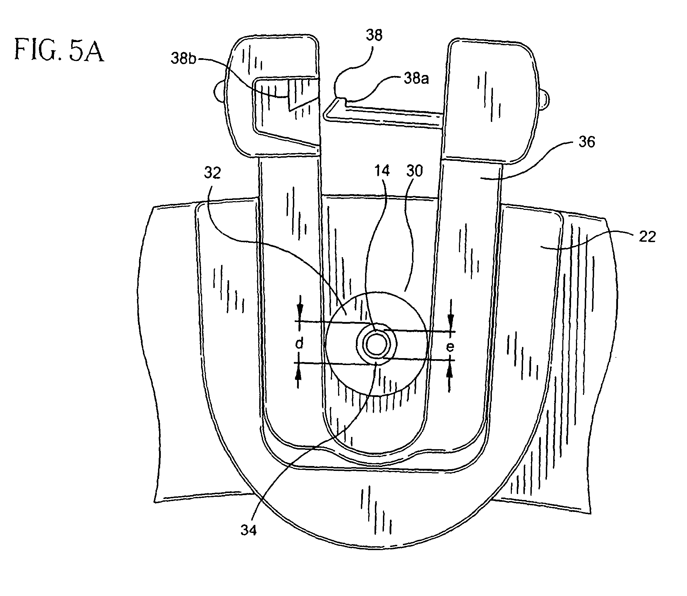

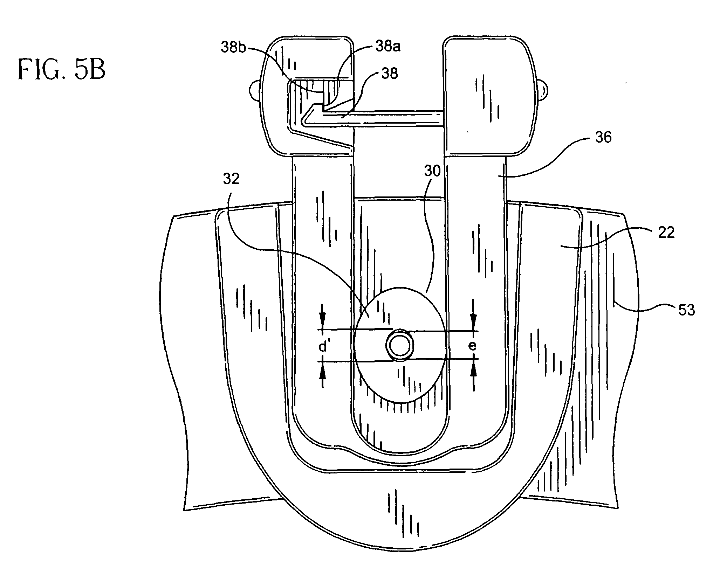

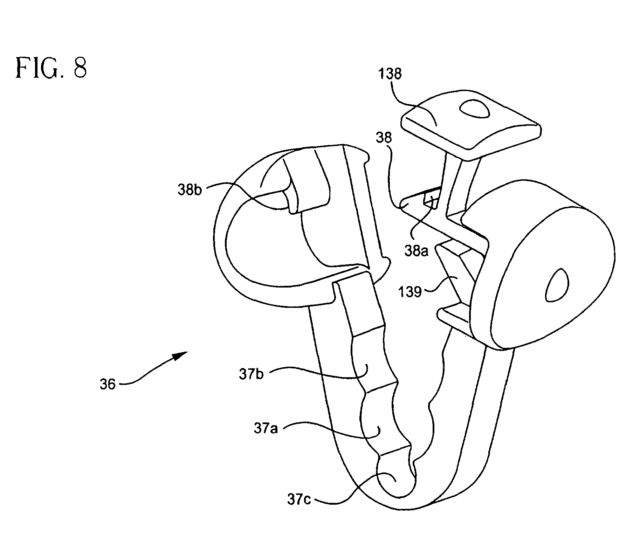

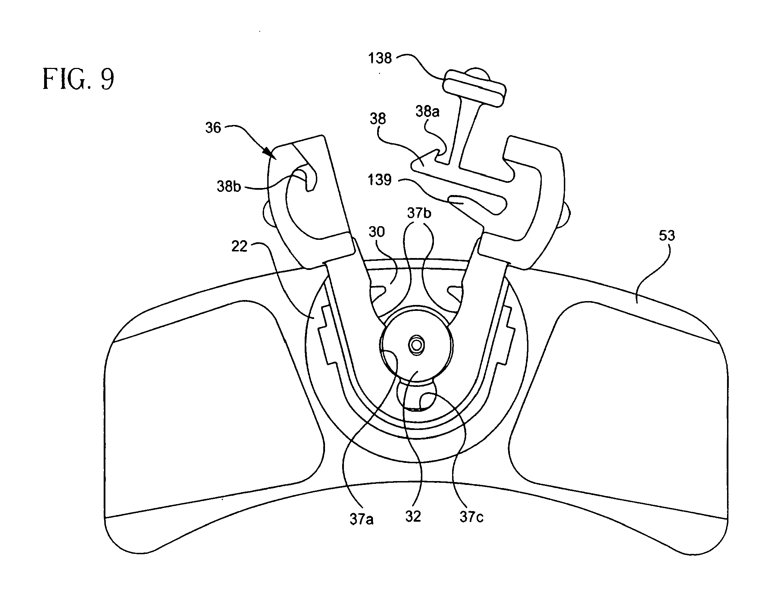

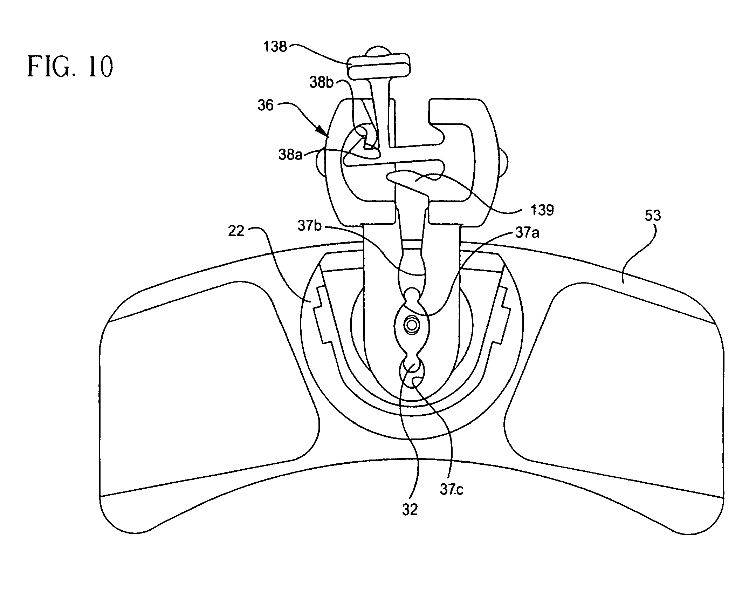

Hub 22 of epidural needle 10 has a clamp 36 with a releasable latch 38 disposed about resilient member 32. Clamp 36 is selectively movable between an open position, best seen in Figs. 5A and 9, wherein inner diameter "d" of resilient member opening 34 is substantially unaffected, and a clamp/latch position, best seen in Figs. 5B and 10, wherein clamp 36 causes a strain to resilient member 32 and thereby reduces the inner diameter of opening 34 through the resilient member. Clamp 36 has a generally V or U-shaped configuration where the apex is formed as a living hinge to allow clamp 36 to move between the open and clamp/latch positions. Preferably clamp 36 is resilient and biased toward the open position. As seen in Figs. 8 - 11, the inner surfaces of each leg of clamp 36 can be formed with a main radiused portion 37a having at least one radius that is substantially the same as the radius of the outer surface of resilient member 32. Preferably, the inner surfaces of each leg of clamp 36 is also formed with an upper radiused portion 37b and a lower radiused portion 37c. Upper radiused portion 37b and lower radiused portion 37c provide space into which portions of resilient member 32 may extend when clamp 36 is moved to the clamp position. Compare FIG. 9 and FIG. 10. This configuration reduces the force needed to move clamp 36 to the clamp/latch position.

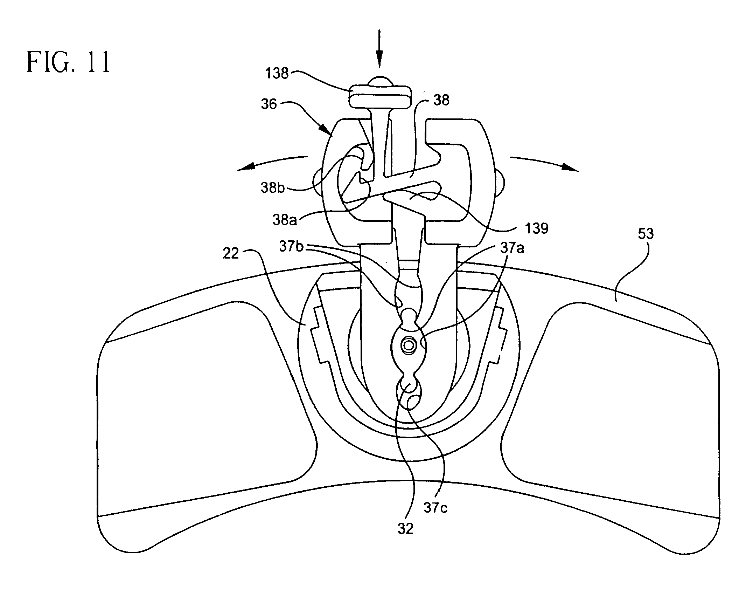

Latch 38 releasably retains clamp 36 in the larch position. Latch 38 extends from one leg of clamp 36 and includes a shoulder 38a that engages a shoulder 38b formed on the other leg of clamp 36. When the two legs of clamp 36 are squeezed together so that shoulder 38a engages shoulder 38b, clamp 36 is held in the latch position. When shoulders 38a and 38b are disengaged, the resiliency of clamp 36 allows the legs of clamp 36 to move apart so that clamp 36 returns to the open position. An upstanding push tab 138 extends upwardly from latch 38. See Figs. 8 - 11. Push tab 138 can be pushed downwardly in order to facilitate the disengagement of shoulders 38a and 38b. As shown in FIGS. 8 - 11, push tab 138 is oriented such that it moves in a direction perpendicular to the longitudinal axis of epidural needle 10 to disengage shoulders 38a and 38b. Since there is no longitudinal component to this motion, it does not affect the relative longitudinal relationship between epidural needle 10 and spinal needle 14. A support leg 139 can be located below latch 38 to support latch 38 and prevent push tab 138 from being used to break latch 38 if excessive force is applied downwardly to push tab 138 when clamp 36 is opened. See Fig. 11.

Thus, a practitioner using epidural needle 10 to position spinal needle 14 with an outside diameter "e" less than the inside diameter "b" of hollow bore 12 may freely axially move spinal needle 14 within hollow bore 12 with respect to epidural needle 10 and fix a position of a distal point 40 of spinal needle 14 relative to epidural needle 10 by the reduction of inner diameter "d" of opening 34 through resilient member 32 to a diameter "d"' less than outside diameter "e" of spinal needle 14 by movement of clamp 36 to the clamp/latch position. The design of clamp 36 is provided to illustrate the invention. Other designs for clamp 36 which cause sufficient strain on resilient member 32 to reduce inside diameter "b" sufficiently to fix the position of spinal needle 14 may be envisioned.

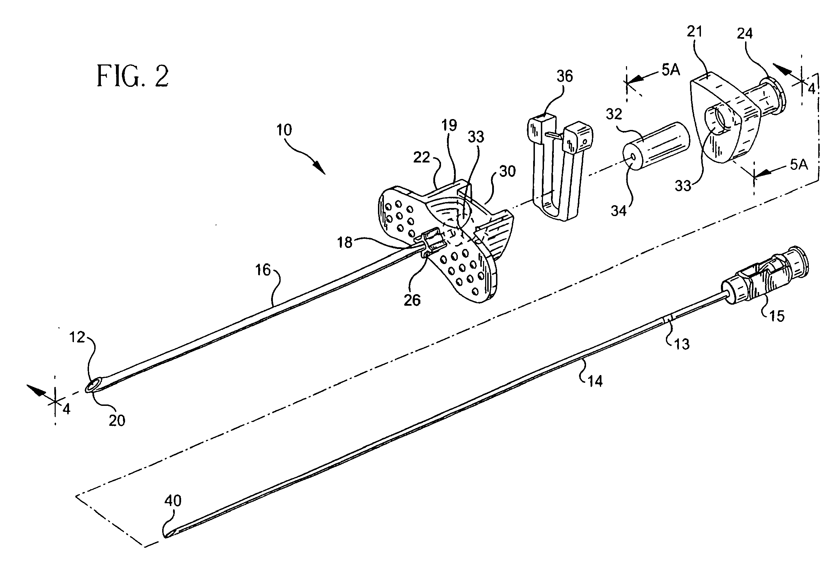

As best seen in Fig. 1, hub 22 of the epidural needle and a hub 51 of stylet 50 preferably are shaped to facilitate the practitioner's handling. For particular applications, hub 15 of spinal needle 14 may also have a similar shape, as shown in Fig. 1, or a more conventional shape as shown in Fig. 2. Hub 22 also preferably includes wings 53, which preferably are fixedly attached to the hub, but may be removable for particular applications. Proximal end 24 of hub 22 also includes an attachment for a fluid handling device, preferably a female luer fitting 25.

Preferably, hub 22 is formed in two portions, a distal portion 19 and a proximal portion 21 that are joined together after placement of resilient member 32 with clamp 36 into cavity 30. Distal portion 19 and proximal portion 21 may be joined together by snap fit, adhesive bonding, solvent bonding, thermal welding, sonic welding or other techniques for fixedly attaching parts formed from thermoplastic materials. Preferably, the parts are joined by ultrasonic welding. Preferably, proximal portion 21 and distal portion 19 each define part of cavity 30 and each includes a recess 33 shaped to form a substantially fluid tight seal about resilient member 32 so that hollow bore 12 of epidural needle 10 is in substantially fluid tight communication with preferred female luer fitting 25. Since resilient member 32 forms a substantially fluid tight seal between bore 12 and female luer fitting 25, epidural needle 10 is fully suitable for any procedure, including, but not limited to, use with a loss of resistance syringe normally practiced with standard epidural needles. In addition, as discussed above epidural needle 10 has the ability to fix the position of a spinal needle with respect to the epidural needle. This versatility of use is not possible with previous variable extension spinal/epidural devices.

Preferably, epidural needle 10 is part of a kit 11 that includes spinal needle 14 and a stylet 50 to occlude hollow bore 12 of the epidural needle. These items are placed in a package 52, illustrated in phantom in Fig. 1. Kit 11 may also include a stylet 17 for spinal needle 14. Kit 11 may also include other items (not shown) in addition to spinal needle 14 and stylet 17, such as gloves, skin preparation materials. medicaments, epidural catheters with connectors, filters and the like for particular applications.

Package 52 is preferably formed from materials substantially resistant to microorganisms, sealed and exposed to conditions suitable to render any microorganisms therein non-viable. Suitable materials for forming package 52 include but are not limited to thermoplastic films, metallic foils, paper, non-wovens as well as combinations of these materials. Suitable conditions for rendering microorganisms non-viable include, but are not limited to, exposure to gaseous agents such as ethylene oxide, vapor phase hydrogen peroxide and the like, and exposure to ionizing radiation such as is generated by electron beam, ultraviolet or gamma radiation.

Resilient member 32 is preferably formed from a resilient elastomeric material. Suitable elastomeric materials include, but are not limited to, natural rubber, synthetic rubber, silicone elastomer, ethylene propylene diene monomer (EPDM) and the like. Preferably, a resilient material is selected with a Shore A durometer between about 30 and 80 to be formed into resilient member 32. In the figures resilient member 32 is shown as a cylinder. However, other elongate shapes also are considered within the scope of the disclosure and may be selected for particular applications.

Suitable materials for forming hub 22 include, but are not limited to, thermoplastic resins such as polypropylene, polyethylene, polycarbonate, polystyrene and the like. Generally, it is preferable to form elongate tube 16 from a metallic material such as a stainless steel. Preferably, elongate tube 16 is fixedly attached to hub 22 by insert molding, but other known methods of bonding including, but not limited to, adhesive bonding and the like may be used for particular applications. Preferably, distal point 20 of epidural needle 10 is formed into a sharpened point suitable for penetrating a patient's tissue.

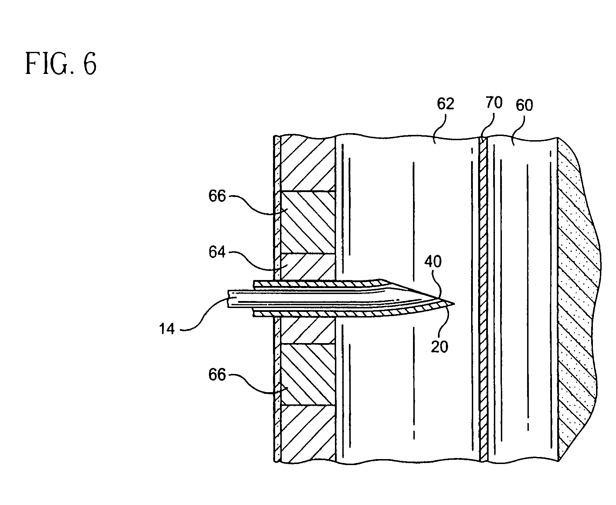

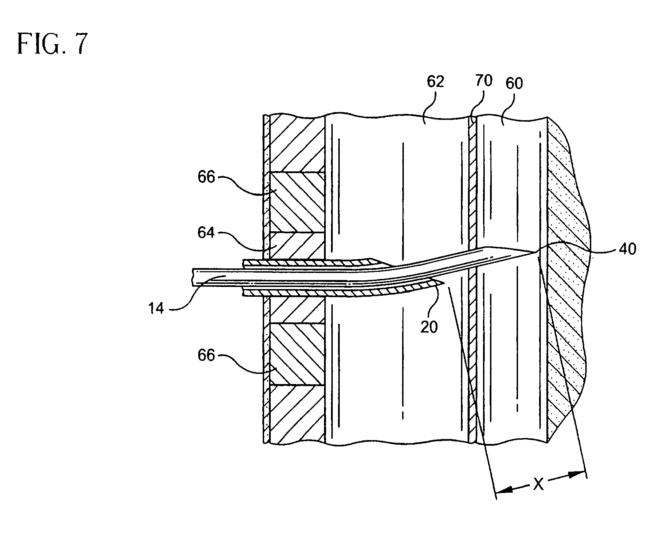

Figs. 6 and 7 illustrate a schematic cross section of a patient's spine 62 and show a method for a practitioner to use epidural needle 10 to position spinal needle 14 in the subarachnoid space 60. This method includes positioning epidural needle 10 in the lumbar region 64 of spine 62 between the vertebrae 66 so that distal point 20 of epidural needle 10 is in close proximity to the dural membrane 70. Preferably, epidural needle bore 12 is occluded by stylet 50 during the penetration of epidural needle 10 through the patient's tissue so that no tissue core is cut, forced into bore 12 and possibly introduced to the epidural space or into the patient's spine by the instillation of the medicament. To complete and confirm the placement of epidural needle 10 in the epidural space, the practitioner withdraws stylet 50 from bore 12 and then may attach a "loss of resistance" syringe containing normal saline solution or air. The practitioner then advances epidural needle 10 while applying pressure to the syringe plunger. Upon penetration into the epidural space, the practitioner perceives a "loss of resistance" to movement of the syringe plunger and the syringe contents are delivered into the now created epidural space. Following this, the practitioner removes the "loss of resistance" syringe and introduces spinal needle 14 into bore 12.

Epidural needle 10 by having resilient member 32 form a substantially air and fluid tight seal between bore 12 of epidural needle 10 and attachment 24 for a fluid handling device, allows the practitioner to use epidural needle 10 as a conventional needle for performing the "loss of resistance test". Earlier variable extension needles such as disclosed in U.S. Patent No. 5,584,820 do not allow such use, because there is no fluid tight seal between the bore of the needle and a fluid attachment.

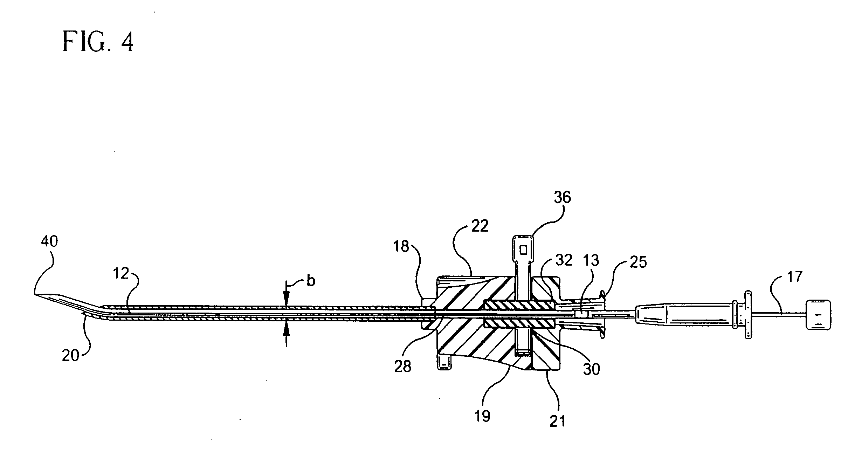

The practitioner preferably leaves clamp 36 in the open position to allow slidable movement of spinal needle 14 through bore 12. Spinal needle 14 preferably has indicia 13 formed thereon by, e.g. etching, to indicate the position of distal point 40 of spinal needle 14 relative to distal point 20 of the epidural needle. Specifically, indicia 13 is located on spinal needle 14 at a distance from distal point 40 that is substantially equal to the overall length of epidural needle 10. In this manner, the practitioner will know when distal point 40 is about to emerge from the distal end of epidural needle 10 as the practitioner advances spinal needle 14 through epidural needle 14. See e.g. FIG. 4. The practitioner advances epidural needle point 20 to close proximity to the dural membrane 70 and advances spinal needle 14 until the distal point 40 penetrates the dural membrane 70 and enters subarachnoid space 60. The practitioner then may move clamp 36 to the clamp position and engage latch 38 to fix the position of spinal needle 14 relative to epidural needle 10 with a projection distance "X". Preferably, latch 38 of clamp 36 is selectively engageable and releasable by the practitioner to accommodate the practitioner's needs during the procedure. Spinal needle 14 may also include a removable stylet 17 to occlude the bore of spinal needle 14 until the practitioner has completed the placement of spinal needle 14. Once placement of spinal needle 14 in the subarachnoid space 60 is achieved and confirmed, the practitioner then may attach a fluid handling device such as a syringe to spinal needle 14 and instill the medicament into the subarachnoid space 60.

The table below relates standard needle gauge sizes to the inner and outer diameter of hypodermic tubing used for forming the needles described above.

**Table of Hypodermic Tubing Nominal Sizes**

| Gauge | Outside Diameter (mm) | Inside Diameter (mm) |
|---|---|---|
| 30 | 0.30 | 0.18 |
| 29 | 0.33 | 0.20 |
| 28 | 0.36 | 0.20 |
| 27 | 0.40 | 0.25 |
| 26 | 0.46 | 0.30 |
| 25 | 0.51 | 0.30 |
| 24 | 0.56 | 0.36 |
| 23 | 0.64 | 0.38 |
| 22 | 0.71 | 0.46 |
| 21 | 0.82 | 0.56 |
| 20 | 0.90 | 0.65 |
| 19 | 1.08 | 0.80 |
| 18 | 1.27 | 0.96 |
| 17 | 1.50 | 1.17 |
| 16 | 1.65 | 1.32 |

Referring to the table of nominal needle gauge sizes above, the preferred needle set includes a twenty-seven gauge spinal needle 14 slidably fit within an eighteen gauge epidural needle 10. Alternatively, a combination of a twenty-five gauge spinal needle 14 and a seventeen gauge epidural needle 10, a twenty-seven gauge spinal needle 14 and a seventeen gauge epidural needle 10 or a twenty-nine gauge spinal needle 14 and an eighteen gauge epidural needle 10 or other similar combinations may be preferred for particular applications. The larger number gauge size (smaller outside diameters) combinations are often preferred for patients of smaller stature or for pediatric applications. Spinal needles 14 having gauge sizes between about twenty-two gauge and twenty-nine gauge are preferred by most practitioners for most applications. Useful needle length ranges accommodative of most patient statures include epidural needle 10 having an effective penetration length between about 8 cm to about 9 cm and spinal needle 14 having a sufficient length so that projection distance ("X") of spinal needle point 40 beyond epidural needle point 20 when spinal needle 14 is fully seated in epidural needle 10 is between about 13.5 mm to about 16.5 mm. For particular applications other lengths of both the spinal and epidural needles may be preferred. In general, consideration of a number of factors including, but not limited to, the desired spinal needle projection ("X") range and the patient stature range should be considered when selecting design parameters including, but not limited to, gauge sizes, needle lengths and the particular configuration of the projection adjustment mechanism for the epidural needle. Numerous other combinations of these design parameters beyond those described in this disclosure may be envisioned.

Epidural needle 10 provides practitioners an improvement in their ability to deliver medicaments to the subarachnoid space. Since epidural needle 10 provides a fluid tight and unrestricted path between bore 12 and attachment 24 as long as clamp 36 is not engaged, the epidural needle is suitable for any normal procedure that may be desired by the practitioner. The epidural needle of the invention then allows the practitioner to fix the position of the spinal needle with respect to the epidural needle. The epidural needle of the invention in combination with a standard spinal needle or a preferred spinal needle having a hub shape similar to the preferred shape of the epidural needle hub is easy to use and allows the practitioner more control of the penetration of the dural membrane than currently available needle sets. By providing the practitioner with more control, the needle set substantially reduces the chance of adverse effects on the patient receiving the treatment.

## Claims

1. An epidural needle (10), comprising:
an elongate tube (16) defining a longitudinal axis having a proximal end (18), a distal end (20) and an axial hollow bore (12) having an inside diameter therethrough;
a hub (22) having a proximal end (24), a distal end (26) and an open passageway (28) therethrough, said hub (22) being attached to said elongate tube (16) so that said hollow bore (12) of said elongate tube is in fluid communication and substantial axial alignment with said open passageway (28), said hub further having a cavity (30) therein disposed between said proximal end (24) and said distal end (26) of said hub;
a resilient member (32) having an opening (34) therethrough defining an inner diameter and disposed in said cavity (30) so that said opening is substantially axially aligned and in fluid communication with said open passageway (28); and
a clamp (36) selectively movable between an open position wherein said inner diameter of said resilient member (32) is substantially unaffected and a clamp position wherein said clamp causes a strain to at least a portion of said resilient member (32) thereby reducing said inner diameter of said opening (34) through at least a portion of said resilient member;
**characterised in that** the clamp (36) includes a pair of legs defining at least one radiused portion (37a) therein, which has a radius substantially the same as a radiused portion defined by the resilient member (32), and the pair of legs defines a second radiused portion (37b, 37c) adjacent to the at least one radiused portion (37a).

2. The epidural needle of claim 1 wherein at least a portion of said clamp (36) projects outwardly from said hub (22) to facilitate the practitioner's selective movement of said clamp (36) between said open position and said clamp position.

3. The epidural needle of claim 2 wherein said portion of said clamp (36) that projects outwardly from said hub further includes a releasable latch (38) for selectively retaining said clamp in said clamp position.

4. The epidural needle of claim 3 further including a push tab (138) extending away from the releasable latch (38) to facilitate unclamping said clamp (36) from said clamp position.

5. The epidural needle of claim 4 wherein the push tab (138) is oriented for movement perpendicular to the elongate tube (16).

6. The epidural needle of claim 3 further including a support leg (139) that limits movement of the latch (38).

7. A combined spinal epidural needle set comprising the epidural needle of claim 1, 2, or 4 to 6, wherein said clamp has a releasable latch (38) disposed about said resilient member (32); and
a spinal needle (14) having an outside diameter less than said inside diameter of said hollow tube (16) disposed within said hollow bore (12), and wherein a practitioner using said epidural needle to position said spinal needle may freely axially move said spinal needle (14) within said hollow bore (12) with respect to said epidural needle (10) and fix a position of said spinal needle relative to said epidural needle by said reduction of said inner diameter opening through said resilient member (32) to a diameter less than said outside diameter of the spinal needle (14) by movement of said clamp (36) to said clamp position thereby to grasp releasably the spinal needle sufficiently to fix the position of the spinal needle with respect to the epidural needle.

8. The combined spinal epidural needle set of claim 7 wherein the spinal needle (14) includes an indicia (13) formed thereon for providing an indication to the practitioner of the location of the spinal needle with respect to the epidural needle.

## Patentansprüche

1. Epiduralnadel (10), die aufweist:
ein längliches Rohr (16), das eine Längsachse mit einem proximalen Ende (18), einem distalen Ende (20) und einem axialen hohlen Loch (12) mit einem Innendurchmesser dort hindurch definiert;
eine Nabe (22) mit einem proximalen Ende (24), einem distalen Ende (26) und einem offenen Durchgang (28) dort hindurch, wobei die Nabe (22) am länglichen Rohr (16) befestigt ist, so dass das hohle Loch (12) des länglichen Rohres in Fluidverbindung und wesentlicher axialer Ausrichtung mit dem offenen Durchgang (28) ist, wobei die Nabe außerdem einen Hohlraum (30) darin aufweist, der zwischen dem proximalen Ende (24) und dem distalen Ende (26) der Nabe angeordnet ist;
ein elastisches Element (32) mit einer Öffnung (34) dort hindurch, die einen Innendurchmesser definiert und im Hohlraum (30) angeordnet ist, so dass die Öffnung im Wesentlichen axial ausgerichtet und in Fluidverbindung mit dem offenen Durchgang (28) ist; und
eine Klemme (36), die selektiv zwischen einer offenen Position, bei der der Innendurchmesser des elastischen Elementes (32) im Wesentlichen unbeeinflusst ist, und einer Klemmposition beweglich ist, bei der die Klemme eine Beanspruchung an mindestens einem Abschnitt des elastischen Elementes (32) hervorruft, wodurch der Innendurchmesser der Öffnung (34) durch mindestens einen Abschnitt des elastischen Elementes verringert wird;
**dadurch gekennzeichnet, dass** die Klemme (36) ein Paar Schenkel umfasst, die mindestens einen abgerundeten Abschnitt (37a) darin definieren, der einen Radius aufweist, der im Wesentlichen der gleiche ist wie ein abgerundeter Abschnitt, der durch das elastische Element (32) definiert wird, und dass das Paar der Schenkel einen zweiten abgerundeten Abschnitt (37b, 37c), benachbart dem mindestens einen abgerundeten Abschnitt (37a), definiert.

2. Epiduralnadel nach Anspruch 1, bei der mindestens ein Abschnitt der Klemme (36) nach außen aus der Nabe (22) vorsteht, um die selektive Bewegung der Klemme (36) zwischen der offenen Position und der Klemmposition durch den Mediziner zu erleichtern.

3. Epiduralnadel nach Anspruch 2, bei der der Abschnitt der Klemme (36), der nach außen aus der Nabe vorsteht, außerdem eine lösbare Verriegelung (38) für das selektive Halten der Klemme in der Klemmposition einschließt.

4. Epiduralnadel nach Anspruch 3, die außerdem eine Drucknase (138) umfasst, die sich von der lösbaren Verriegelung (38) weg erstreckt, um das Freigeben der Klemme (36) aus der Klemmposition zu erleichtern.

5. Epiduralnadel nach Anspruch 4, bei der die Drucknase (138) für eine Bewegung senkrecht zum länglichen Rohr (16) ausgerichtet ist.

6. Epiduralnadel nach Anspruch 3, die außerdem einen Halteschenkel (139) einschließt, der die Bewegung der Verriegelung (38) begrenzt.

7. Kombinierter Spinal-Epiduralnadelset, das aufweist:
die Epiduralnadel nach Anspruch 1, 2 oder 4 bis 6, worin die Klemme eine lösbare Verriegelung (38) aufweist, die um das elastische Element (32) angeordnet ist; und
eine Spinalnadel (14) mit einem Außendurchmesser, der kleiner ist als der Innendurchmesser des hohlen Rohres (16), angeordnet innerhalb des hohlen Loches (12), und wobei ein Mediziner, der die Epiduralnadel verwendet, um die Spinalnadel zu positionieren, die Spinalnadel (14) ungehindert axial innerhalb des hohlen Loches (12) mit Bezugnahme auf die Epiduralnadel (10) bewegen und eine Position der Spinalnadel relativ zur Epiduralnadel durch die Verringerung des inneren Durchmessers der Öffnung durch das elastische Element (32) auf einen Durchmesser kleiner als der Außendurchmesser der Spinalnadel (14) durch die Bewegung der Klemme (36) in die Klemmposition arretieren kann, wodurch die Spinalnadel ausreichend lösbar ergriffen wird, um die Position der Spinalnadel mit Bezugnahme auf die Epiduralnadel zu arretieren.

8. Kombinierter Spinal-Epiduralnadelset nach Anspruch 7, bei dem die Spinalnadel (14) eine Kennzeichnung (13) umfasst, die darauf ausgebildet ist, um dem Mediziner einen Hinweis auf die Position der Spinalnadel mit Bezugnahme auf die Epiduralnadel zu liefern.

## Revendications

1. Aiguille péridurale (10), comprenant :
un tube allongé (16) définissant un axe longitudinal comportant une extrémité proximale (18), une extrémité distale (20) et un perçage axial creux (12) ayant un diamètre intérieur le traversant ;
un moyeu (22) comportant une extrémité proximale (24), une extrémité distale (26) et un passage ouvert (28) le traversant, ledit moyeu (22) étant fixé sur ledit tube allongé (16), de sorte que ledit perçage creux (12) dudit tube allongé est en communication de fluide et aligné de manière pratiquement axiale avec ledit passage ouvert (28), ledit moyeu comportant en outre une cavité (30) agencée entre ladite extrémité proximale (24) et ladite extrémité distale (26) dudit moyeu ;
un élément élastique (32) comportant une ouverture (34) le traversant, définissant un diamètre intérieur et agencé dans ladite cavité (30), de sorte que ladite ouverture est alignée de manière pratiquement axiale et en communication de fluide avec ledit passage ouvert (28) ; et
une pince (36) pouvant être déplacée sélectivement entre une position ouverte, dans laquelle ledit diamètre intérieur dudit élément élastique (32) n'est pratiquement pas affecté, et une position de serrage, dans laquelle ladite pince applique une contrainte à au moins une partie dudit élément élastique (32), réduisant ainsi ledit diamètre intérieur de ladite ouverture (34) traversant au moins une partie dudit élément élastique ;
**caractérisée en ce que** la pince (36) englobe une paire de branches définissant au moins une partie arrondie (37a) ayant un rayon pratiquement identique à celui d'une partie arrondie définie par l'élément élastique (32), la paire de branches définissant une deuxième partie arrondie (37b, 37c) adjacente à la au moins une partie arrondie (37a).

2. Aiguille péridurale selon la revendication 1, dans laquelle au moins une partie de ladite pince (36) déborde vers l'extérieur dudit moyeu (22) pour faciliter le déplacement sélectif de ladite pince (36) par le médecin entre ladite position ouverte et ladite position de serrage.

3. Aiguille péridurale selon la revendication 2, dans laquelle ladite partie de ladite pince (36) débordant vers l'extérieur dudit moyeu englobe en outre un verrou à dégagement (28) pour retenir de manière sélective ladite pince dans ladite position de serrage.

4. Aiguille péridurale selon la revendication 3, englobant en outre une patte de poussée (138) s'étendant à l'écart du verrou à dégagement (38) pour faciliter le desserrage de ladite pince (36) de ladite position de serrage.

5. Aiguille péridurale selon la revendication 4, dans laquelle la patte de poussée (138) est orientée en vue d'un déplacement perpendiculaire par rapport au tube allongé (16).

6. Aiguille péridurale selon la revendication 3, englobant en outre une branche de support (139) limitant le déplacement du verrou (38).

7. Set combiné d'aiguille spinale/péridurale comprenant l'aiguille péridurale selon les revendications 1, 2 ou 4 à 6, la pince comportant un verrou à dégagement (38) agencé autour dudit élément élastique (32) ; et
une aiguille spinale (14) ayant un diamètre extérieur inférieur audit diamètre intérieur dudit tube creux (16), agencée dans ledit perçage creux (12), un médecin utilisant ladite aiguille péridurale pour positionner ladite aiguille spinale pouvant déplacer librement ladite aiguille spinale (14) dans une direction axiale dans ledit perçage creux (12) par rapport à ladite aiguille péridurale (10) et fixer une position de ladite aiguille spinale par rapport à ladite aiguille péridurale en réduisant ledit diamètre intérieur de l'ouverture traversant ledit élément élastique (32) à un diamètre inférieur audit diamètre extérieur de l'aiguille spinale (14), par déplacement de ladite pince (36) vers ladite position de serrage, pour saisir ainsi de manière amovible l'aiguille spinale, de manière suffisante pour fixer la position de l'aiguille spinale par rapport à l'aiguille péridurale.

8. Set combiné d'aiguille spinale/péridurale selon la revendication 7, dans lequel l'aiguille spinale (14) englobe un repère (13) qui y est formé pour indiquer au médecin l'emplacement de l'aiguille spinale par rapport à l'aiguille péridurale.
